# EUROPEAN PATENT APPLICATION

(11) **EP 4 726 935 A1**
(43) Date of publication of application: **15.04.2026**
(21) Application number: 25207086.7
(22) Date of filing: 07.10.2025
(51) Int. Cl.: H01R 13/62, G04C 3/00, H01F 7/02, A61B 5/00

(54) **ELECTRIC CONNECTION ARRANGEMENT**

(30) Priority: 10.10.2024 FI 20246223
(71) Applicant: Polar Electro Oy, 90440 Kempele (FI)
(72) Inventor: Niemimäki, Mika, 90440 Kempele (FI)
(74) Representative: Kolster Oy Ab

(57) **Abstract**

An electronic connection arrangement comprising a frame, at least one electric contact arranged within the frame for an electric connection; and at least a first and a second magnet forming a first magnetic pair arranged within the frame. First poles of the first and second magnet having the same polarity are arranged within the frame such that magnetic fields of the first poles interact, and wherein a second pole of the first magnet is directed away from the frame to be coupled with a magnetic counterpart for positioning and locking the electric contact with its counterpart.

## Description

### TECHNICAL FIELD

The invention relates to a field of electric connection arrangements.

### TECHNICAL BACKGROUND

The electric connection arrangement may refer to a connection interface between a connector and an electronic apparatus, for example. The known connection interface solutions have some drawbacks. The drawbacks often relate to forming a firm and reliable connection between the connector and the electronic apparatus, or interferences caused by the connection interfaces, for example.

Hence, a more sophisticated solution for the electric connection arrangement is needed to alleviate the above-mentioned drawbacks.

### BRIEF DESCRIPTION

The present invention is defined by the subject matter of the independent claim.

Embodiments are defined in the dependent claims.

The embodiments and features, if any, described in this specification that do not fall under the scope of the independent claim are to be interpreted as examples useful for understanding various embodiments of the invention.

### BRIEF DESCRIPTION OF THE DRAWINGS

In the following the invention will be described in greater detail by means of preferred embodiments with reference to the attached drawings, in which
Figures 1A and 1B illustrate an electric connection arrangement according to embodiments of the invention;
Figures 2A and 2B illustrate magnets used in the electric connection arrangement according to embodiments of the invention;
Figure 3 illustrates a magnetic pair used in the electric connection arrangement according to an embodiment of the invention; and
Figure 4 illustrates the electric connection arrangement according to embodiments of the invention.

### DETAILED DESCRIPTION OF THE INVENTION

The following embodiments are exemplifying. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments.

Embodiments of the invention relate to an electric connection arrangement of electronic apparatuses. The electric connection arrangement may be a physical connection interface between a connector and the electronic apparatus, for example. The connector may be coupled with a cable. The interface may be used for charging the electronic apparatus and/or for transferring data, for example. The electronic apparatus may be a mobile device such a smart phone, a navigator, or a wearable biosignal measuring device that comprises components that are sensitive for an external magnetic field. In the content of this application the invention is applied in the field of the wearable biosignal measuring devices, but as descried above, it is just one example, and the invention may be applied in many other electrical apparatuses as well. The wearable biosignal measuring device may be a wearable training computer configured to carry out measurements during a physical exercise performed by a user. The warble training computer may comprise a rechargeable battery, and therefore the wearable training computer comprises an interface for a connector (with a cable) for charging. The interface may also be used for transferring data, for example. The wearable training computer may be a portable system attachable to the user's body. The wearable training computer is configured to measure physiological training data from the user's performance during the physical exercise and to output the training data to the user via a user interface of the training computer and/or via a user interface of another apparatus. The wearable training computer may further comprise an apparatus configured to be attached to the object. Such an apparatus may comprise an attachment structure designed and arranged to receive the training computer in a fixed, integrated, or detachable manner and to attach the training computer to the object. The attachment may be realized by a band that may be designed to encircle the object such that the band is attached around the object. The band may comprise locking parts at ends of the band where the locking parts form mutually counterparts such as a buckle and a catch. The locking parts may fix the band around the object as is commonly known in the field of wristwatches, wrist computers etc. Other forms of attachment of wearable devices are equally possible, e.g. the training computer may be integrated or attached to a garment such as a shirt, pants or harness. In other embodiments, the attachment structure may be arranged to attach the apparatus to the device such as the bicycle. In such embodiments, the apparatus may be or may be comprised in a bike computer, for example.

The magnets are commonly used for positioning and coupling the connector with the apparatus in physical connections. Using of the magnets may cause also some issues. For example, the magnetic field(s) of the magnets may disturb the electric apparatus. The wearable training computer comprises sensitive components that are sensitive to the external magnetic field(s). The external magnetic field may cause magnetization of some parts of the wearable training computer, and/or the magnetically sensitive component(s) may be disturbed. For example, the wearable training computer may comprise a magnetometer that may be interfered by the magnetic field(s) of the coupling magnet(s). The magnetometer may be a one, two or three axis electrical component/sensor designed to measure magnetic field of the earth, for example. Due to the fact that strength of the magnetic field of the Earth is very small, the magnetic field of the external magnet (for example such as placed on the connecter) set nearby may disturb magnetic measurements of the magnetometer. The external magnet can also magnetize the materials or components nearby the magnetic sensor so that although the external magnet is removed, the magnetic effect may still stay there. This may lead to situations in which extra calibrations are needed for the wearable training computer to remove the issue. By placing the magnetometer as far as possible from the magnet(s) may alleviate the issue but it cannot reduce the interferences enough since an area in the wearable training computer is very limited. The invention provides a solution that remarkably alleviates the above-mentioned drawbacks. The wearable training computer is normally used without the recharging cable. The above-described issue may arise when the recharging cable with magnet(s) is connected to the computer. The strong magnetic field of the magnet(s) will cause extending of the magnetic field(s) around the whole area of the wearable training computer. The magnetically sensitive parts and components in the computer may face the strong magnetic field as well to may be, at least weakly, magnetized. This same effect/issue will also happen when removing the recharging cable with magnet(s).

Especially bringing the magnet part(s) close to the computer and removing the magnet part(s) away from the computer may disturb and magnetize the magnetically sensitive parts of the computer. To reduce the effect/issue the solution using "opposed magnets" according to the invention reduces remarkably the magnetic field(s) of the magnets on the side of the magnets and thus reduces the magnetization effect in the vicinity of the connector area (connection interface). Thus, the invention provides the solution that reduces or even totally remove the need for recalibration of the magnetometer after using the recharging cable in contact with the computer.

According to a first aspect of the invention, there is provided an electric connection arrangement comprising a frame, at least one electric contact arranged within the frame for an electric connection, and at least a first and a second magnet forming a first magnetic pair arranged within the frame, wherein first poles of the first and second magnets having the same polarity are arranged such that magnetic fields of the first poles interact, and wherein a second pole of the first magnet is directed away from the frame to be coupled with a magnetic counterpart for positioning and locking the electric contact with its counterpart.

As described above, the electric connection arrangement (also connection arrangement later in this application) may refer to the connection interface used between the connector and the electronic apparatus. A first part of the connection arrangement may be arranged in the connector and a second part of the connection arrangement may be arranged in the electronic apparatus, and the first and the second part of the connection arrangement can be connected to provide an electrical connection between the connector and the apparatus, for example, for charging the apparatus. The connection arrangement according to the invention may be used on the connector side or the apparatus side. For example, the connection arrangement of the invention may be arranged within the connector and its counterpart is then on the apparatus side. The counterpart of the connection arrangement may not fall under the scope of the invention but nevertheless it is useful and important for understanding various embodiments of the invention.

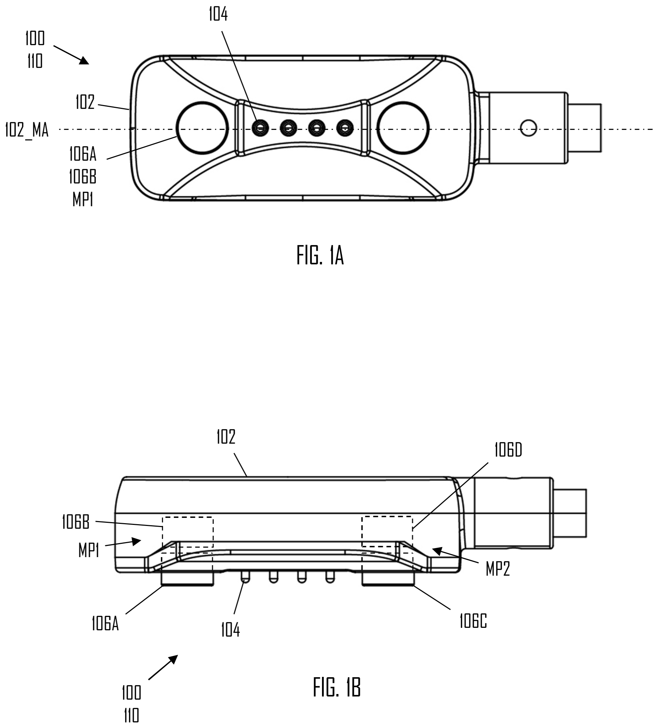

Referring to Figures 1A and 1B, the connection arrangement 100 comprises the frame 102. The frame provides a mechanical structure at least for the components of the arrangement. The frame may be made of plastics or metal, for example.

Still referring to Figures 1A and 1B, the connection arrangement 100 further comprises the electric contact 104 for creating the electric connection with its counterpart. The electric contact is conductive and is arranged to be connected with its counterpart, like another electric contact. The electric contact and its counterpart may be a plug and socket type of structure. Then the electric contact in the connection arrangement may be a pin, for example. The electric contact and its counterpart may also be just a conductive surface that are coupled together to create the electrically conductive connection. The electric contact(s) may be used for conducting electricity (charging of the apparatus) and/or transferring data, for example.

Still referring to Figures 1A and 1B, the connection arrangement further comprises at least the first and the second magnet 106A, 106B to form the first magnetic pair MP1. The first and the second magnet 106A, 106B are arranged within the frame. The frame provides the mechanical structure for holding the magnets in the desired position in relation to each other. The first and second magnet may be arranged within the frame such that the first poles of the first and second magnets that have the same polarity can interact. Then a north pole is interacting with another north pole (N/N), or a south pole is interacting with another south pole (S/S). The second pole of the first magnet is arranged, at least partly, pointing away from the frame such that it can be connected with its counterpart for positioning and holding the electric contact with its counterpart. The counterpart of the second pole may be another magnet or a magnetic material like a magnetic metal, for example. Hence, the second pole of the first magnet ensures that when it is coupled with its counterpart, the electric contact is in the right position in relation to its counterpart and is also held in connection with it for enabling electrical connection for charging or transferring data, for example. The magnets may be arranged inside the frame such that also the second pole of the first magnet is inside the frame and may not be visible. The second pole of the first magnet may also be visible. Anyway, the second magnet and the first pole of the first magnet may be inside the frame may not be visible.

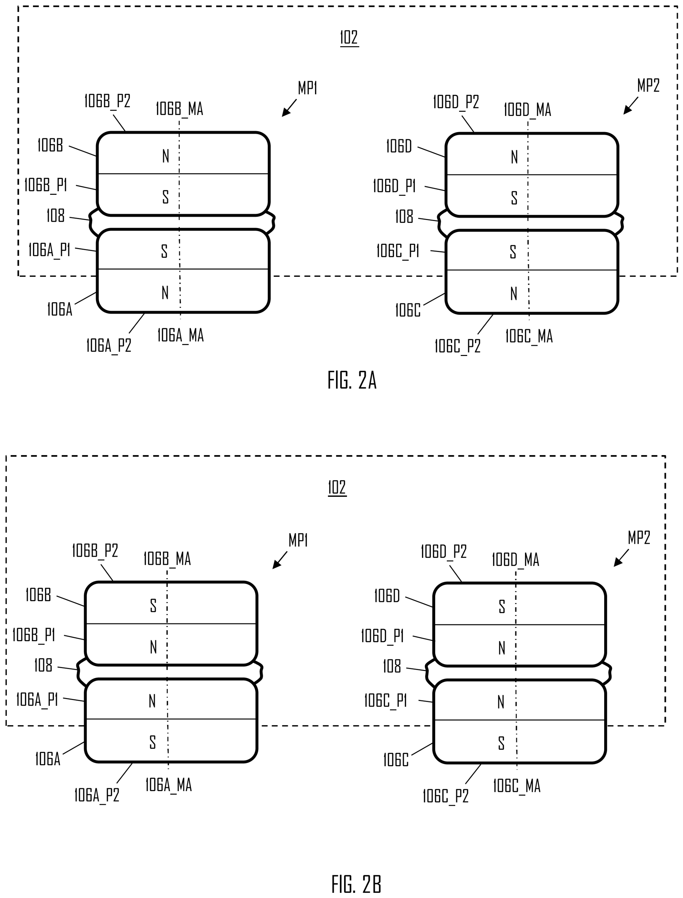

Referring now to Figures 2A and 2B, the first pole of the fist magnet 106A_P1 has the same polarity as the first pole of the second magnet 106B_P1. In example of Figures 2A, the first poles are south S, and in the example of Figure 2B the first poles are north N. The main idea of the inventio is to set the poles having the same polarity in the vicinity of each other, the polarity itself is not so important. The fist poles of the fist and the second magnets are arranged so close to each other that their magnetic fields overlap at least partly. Then the first poles can interact. When the poles having the same polarity are arranged to interact, they try to repel each other. The first poles may be arranged so close to each other as possible to get the strong repelling effect. This reduces the magnetic fields especially on sides of the field(s), and this also reduces interferences in the electrical apparatus and its sensitive components. The arrangement may also weaken strength of the connection of the second pole of the first magnet with its counterpart, but effect is very minor. This may also be compensated by using more powerful magnets, for example. Hence, the magnetic pair comprises a coupling magnet (the first magnet) and an interference elimination (reducing) magnet (the second magnet).

Referring to Figure 1B, in an embodiment, the arrangement further comprises a third and a fourth magnet 106C, 106D forming a second magnetic pair MP2 arranged within the frame 102. First poles of the third and the fourth magnet 106C_P1, 106D_P1 having the same polarity are arranged within the frame 102 such that magnetic fields 106C_P1, 106D_P1 interact. A second pole of the third magnet is directed away from the frame 102 to be coupled with a magnetic counterpart for positioning and locking the electric contact 104 with its counterpart. Hence, the structure and functioning of the second magnetic pair is substantially the same as it is with the first magnetic pair described above. Two magnetic pairs enable more robust and reliable coupling with the counterpart(s). The positioning of the electric contact(s) may be more accurate and coupling between the electric contact(s) and the counterpart(s) may be stronger.

Referring now to Figures 2A and 2B, in an embodiment, the first and second magnet 106A, 106B is arranged side by side within the frame 102 such that the first poles 106A_P1, 106B_P1 are towards each other. Also, the third and fourth magnets 106C, 106D are arranged side by side within the frame 102 such that the first poles 106C_P1, 106D_P1 are towards each other. The term "towards each other" refers to a position in which the first poles of the first and the second magnets are, at least partly, facing each other. Then the repelling effect of the first poles (having the same polarity) is stronger.

Still referring to Figures 2A and 2B, in an embodiment, a magnetic axis of the first and second magnet 106A_MA, 106B_MA are parallel, and a magnetic axis of the third and fourth magnet 106C_MA, 106D_MA are parallel. Hence, the magnets in the first and second magnetic pair are, at least partly, in the same position within the frame. Also surfaces of the first poles may then be parallel in the first and also in the second magnetic pair.

Still referring to Figures 2A and 2B, in an embodiment, the magnetic axis of the first and second magnet 106A_MA, 106B_MA are aligned. Also, the magnetic axis of the third and fourth magnet 106C_MA, 106D_MA are aligned. Hence, the first poles of the first and second magnet in the first magnetic pair, and the first poles of the third and the fourth magnet in the second magnetic pair are aligned. Then the first poles are pointing (exactly) towards each other that provides the strongest repelling effect that may also alleviate the interferences optimally.

In an embodiment, as illustrated in Figures 2A and 2B, the first and second magnets 106A, 106B and/or the third and fourth magnets 106C, 106D are coupled together by a coupling element 108. The coupling element keeps the magnets of each magnet pair in the desired position in relation to each other. For example, the coupling element may couple the first and second magnets and/or the third and fourth magnets together and ensures that the first magnet stays also in the correct position in relation to the second magnet, and the third magnet stays in the correct position in relation to the fourth magnet. In an embodiment, the coupling element is the frame. In another embodiment, the coupling element is a separate component. Each magnet pair may comprise the coupling element, hence the arrangement may comprise two coupling elements in the case of two magnetic pairs. The coupling element may be, for example, a mechanical and/or chemical component used for coupling magnets of the magnetic pairs together.

In an embodiment, the coupling element 108 comprises an adhesive (glue). Hence, the first and second magnets in the first magnetic pair and the third and fourth magnets in the second magnetic pair may be adhered (glued) together. The adhesive may be dispensed between the first poles of the magnets in each magnetic pair that may be arranged to face each other, for example. Then the first poles of the first and second magnets are adhered together as well as the first poles of the third and fourth magnets as illustrated in Figures 2A and 2B. By adjusting the amount of the adhesive between the first poles, a distance between them in the first and second magnetic pairs may be adjusted, for example. The magnets may be adhered together before assembled to the arrangement. The coupling element may comprise the mechanical component like the frame and further the chemical component like the adhesive, for example.

In an embodiment, the first, the second, the third and/or the fourth magnet 106A, 106B, 106C, 106D comprises a permanent magnet. The term "permanent magnet" means that the magnets are made of materials where the magnetic field is generated by an internal structure of the material itself and the magnets can maintain their magnetism for a long time. In an embodiment, the first, the second, the third and/or the fourth magnet comprises an electromagnet. The electromagnet may further comprise a coil for enabling disabling magnetism of the electromagnets.

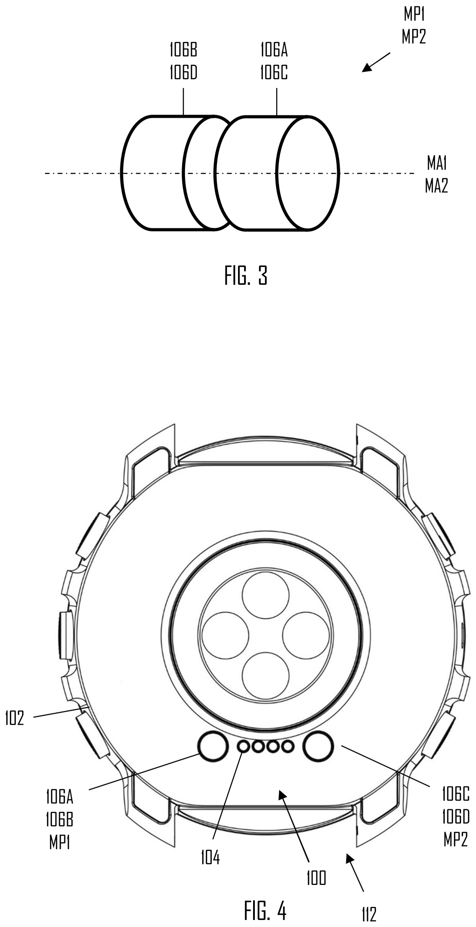

Referring to Figure 3, in an embodiment, a shape of the first, the second, the third and/or the fourth magnet 106A, 106B, 106C, 106D is cylindrical. The shape may also be square or rectangle, for example. The magnets used in the arrangement may be axially magnetized, for example. The magnetic axis MA1, MA2 may refer to a longitudinal middle axis of the cylindrical magnet as illustrated in Figure 3.

In an embodiment, the first, second, third and/or fourth magnet comprises the electric contact. In other words, the magnet itself may form the electric contact. Hence, the magnets that are coupled with the counterpart may form a conductive connection for charging or transferring data.

Referring now to Figures 1A and 1B, in an embodiment, the at least one electric contact 104 is arranged substantially between the first and second pair of the magnets MP1, MP2. The arrangement may comprise a plurality of electric contacts that are arranged between the first and second magnetic pairs. The electric contacts may be on the line and the first magnetic pair is arranged on a first end of the line and the second magnetic pair is arranges on a second end of the line, for example. The first and second magnetic pairs and the electric contacts may be on the same line as illustrated with a centreline 102_MA in Figure 1A. The centreline 102_MA may be a centreline of the frame 102, for example. Then the first and second magnetic pairs and the electric contacts are aligned on the same line and are substantially in the middle of the frame as illustrated in Figure 1A.

Referring now to Figures 2A and 2B, in an embodiment, the polarity of the first poles of the first, second, third and fourth magnet 106A_P1, 106B_P1, 106C_P1, 106D_P1 is the same. As described, the first poles of the first and second magnets and the third and fourth magnets arranged to interact. The polarity of these poles may be the same. In an example of Figure 2A, all the first poles have the polarity south (S), and in an example of Figure 2B all the first poles have the polarity north (N). It is not important is the polarity north or south, the most important thing is that the polarity of the first poles that are arranged to interact within the first and second magnetic pairs is the same to get the desired effect.

According to a second aspect of the invention, there is provided a connector 110 comprising all or some of the features of the electric connection arrangement described above. This embodiment is illustrated in Figures 1A and 1B, for example. Hence, the electric connection arrangement may be integrated within the connector assembly. The connector assembly may be configured to be coupled by the electric connection arrangement with the electronic apparatus like a biosignal measuring device, for example. The biosignal measuring device may comprise the sensitive components like the magnetometer that may be interfered by the coupling magnets. The connector assembly may be coupled with a cable, for example. The frame 102 of the arrangement 100 may be a body of the connector 110. Hence, the first and/or the second magnetic pair as well as the electric contacts may be arranged within the body of the connector and the counterparts for the magnet(s) and the electric contact(s) may be arranged within the electronic apparatus. The connector assembly may be used for charging the electronic apparatus or transferring data, for example.

According to a third aspect of the invention, there is provided a biosensing measuring device 112 comprising all or some of the features of the electric connection arrangement 100 described above. This embodiment is illustrated in Figure 4. Hence, the electric connection arrangement may be integrated within the biosensing measuring device. The biosignal measuring device may be wearable like the wearable training computer, for example. The wearable training computer 112 may comprise the sensitive components like the magnetometer 114 that may be interfered by the coupling magnets. The frame 102 of the arrangement 100 may be a casing of the biosensing measuring device 112, for example. Hence, the first and/or the second magnetic pair as well as the electric contacts may be arranged within the casing of the biosensing measuring device and the counterparts for the magnet(s) and the electric contact(s) may be arranged within the connector assembly. The connector assembly may be used for charging the biosensing measuring device or transferring data, for example.

As described above, the electric connection arrangement according to the invention may be implemented on the connector side or on the apparatus side. If the connection arrangement is implemented into the connector, its counterpart(s) is arranged in the apparatus. If the connection arrangement is implemented into the apparatus like the wearable training computer, its counterpart(s) is arranged in the connector. As described above, by arranging two magnets such that the poles having the same polarity can interact, the interferences caused by the coupling magnet reduces remarkably. When the poles having the same polarity are arranged to interact, the magnetic field(s) are formed such that the interferences are reduced. This provides many benefits since the sensitive components of the electric apparatus are on disturbed and, for example, some calibrations may be avoided. The invention is further structurally easy to implement within the connector assembly and/or the electric apparatus.

It will be obvious to a person skilled in the art that, as the technology advances, the inventive concept can be implemented in various ways. The invention and its embodiments are not limited to the examples described above but may vary within the scope of the claims.

## Claims

1. An electric connection arrangement (100) comprising:
a frame (102);
at least one electric contact (104) arranged within the frame (102) for an electric connection; and
at least a first and a second magnet (106A, 106B) forming a first magnetic pair (MP1) arranged within the frame (102), wherein
first poles of the first and second magnet (106A_P1, 106B_P1) having the same polarity are arranged such that magnetic fields of the first poles (106A_P1, 106B_P1) interact, and wherein a second pole of the first magnet (106A_P2) is directed away from the frame (102) to be coupled with a magnetic counterpart for positioning and locking the electric contact (104) with its counterpart.

2. The arrangement (100) of claim 1, wherein the arrangement further comprises a third and a fourth magnet (106C, 106D) forming a second magnetic pair (MP2) arranged within the frame (102),
wherein first poles of the third and the fourth magnet (106C_P1, 106D_P1) having the same polarity are arranged such that magnetic fields of the first poles (106C_P1, 106D_P1) interact, and wherein a second pole of the third magnet (106C_P2) is directed away from the frame (102) to be coupled with a magnetic counterpart for positioning and locking the electric contact (104) with its counterpart.

3. The arrangement (100) of claim 2, wherein the first and the second magnet (106A, 106B) is arranged side by side within the frame (102) such that the first poles (106A_P1, 106B_P1) are towards each other, and/or
the third and fourth magnet (106C, 106D) is arranged side by side within the frame (102) such that the first poles (106C_P1, 106D_P1) are towards each other.

4. The arrangement (100) of any of claims 2 - 3, wherein a magnetic axis of the first and the second magnet (106A_MA, 106B_MA), and/or the third and the fourth magnet (106C_MA, 106D_MA) are parallel.

5. The arrangement (100) of any of claims 2 - 4, wherein the magnetic axis of the first and the second magnet (106A_MA, 106B_MA) and/or the third and the fourth magnet (106C_MA, 106D_MA) are aligned.

6. The arrangement (100) of any claims 2 - 5, wherein the first and the second magnet (106A, 106B) and the third and fourth magnet (106C, 106D) is coupled together by a coupling element (108).

7. The arrangement (100) of claim 6, wherein the coupling element (108) comprises an adhesive

8. The arrangement (100) of any of claims 2 - 7, wherein the first, the second, the third and/or the fourth magnet (106A, 106B, 106C, 106D) comprises a permanent magnet.

9. The arrangement (100) of any of claims 2 - 8, wherein a shape of the first, the second, the third and/or the fourth magnet (106A, 106B, 106C, 106D) is cylindrical.

10. The arrangement (100) of any of claims 2 - 9, wherein the at least one electric contact (104) is arranged substantially between the first and the second pair of the magnets (MP1, MP2).

11. The arrangement (100) of any of claims 2 - 9, wherein the polarity of the first pole of the first, the second, the third and the fourth magnet (106A_P1, 106B_P1, 106C_P1, 106D_P1) is the same.

12. The arrangement (100) of any preceding claim, wherein the arrangement further comprises a connector (110), wherein the frame (102) is a body of the connector (110).

13. The arrangement (100) of any claim 1 - 11, wherein the arrangement further comprises a biosensing measuring device (112), wherein the frame (102) is a casing of the biosensing measuring device (112).

14. The arrangement (100) of claim 13, wherein the biosensing measuring device (112) comprises a magnetometer for detecting a magnetic field of earth.
